# EUROPEAN PATENT SPECIFICATION

(11) **EP 1 105 765 B1**
(45) Date of publication and mention of the grant of the patent: **08.10.2003**
(21) Application number: 99941209.1
(22) Date of filing: 18.08.1999
(51) Int. Cl.: G02B 26/10, A61B 1/00, G02F 1/29

(54) **OPTICAL SCANNING AND IMAGING SYSTEM**
OPTISCHES ABTAST- UND ABBILDUNGSSYSTEM
SYSTEME DE BALAYAGE OPTIQUE ET DE FORMATION D'IMAGE

(30) Priority: 19.08.1998 US 97043 P
(43) Date of publication of application: 13.06.2001
(73) Proprietor: Boston Scientific Limited, St. Michael, Barbados, West Indies (BB)
(72) Inventor: OSTROVSKY, Isaac, Wellesley, MA 02184 (US)
(74) Representative: Crump, Julian Richard John
(86) International application number: US9918716
(87) International publication number: WO00011511

(56) References cited:
- WO-A-92/16865
- WO-A-98/38907
- US-A- 5 498 869
- PATENT ABSTRACTS OF JAPAN vol. 012, no. 125 (P-691), 19 April 1988 (1988-04-19) & JP 62 250427 A (MATSUSHITA ELECTRIC IND CO LTD), 31 October 1987 (1987-10-31)

## Description

The present invention relates to a system for scanning and imaging body tissue. More specifically, the invention relates to a scanning and imaging system that utilizes scanning components without mechanical motion.

A variety of imaging techniques are used for the medical diagnosis and treatment of patients. Ultrasound imaging represents a prevalent technique. Ultrasound uses sound waves to obtain a cross-sectional image of an object. These waves are radiated by a transducer, directed into the tissues of a patient, and reflected from the tissues. The transducer also operates as a receiver to receive the reflected waves and electronically process them for ultimate display.

Another imaging technique is referred to as Optical Coherence Tomography (OCT). OCT uses light. as opposed to sound waves, to obtain a cross-sectional image of tissue. The use of light allows for faster scanning times than occurs in ultrasound technology. The depth of tissue scan in OCT is based on low coherence interferometry. Low coherence interferometry involves splitting a light beam from a low coherence light source into two beams, a sampling beam and a reference beam. These two beams are then used to form an interferometer. The sampling beam hits and penetrates the tissue, or other object, under measurement, and then reflects from the tissue, carrying information about the reflecting points from the surface and the depth of tissue. The reference beam hits a reference reflector, such as, for example, a mirror or a diffraction grating, and reflects from the reference reflector. The reference reflector either moves or is designed such that the reflection occurs at different distances from the beam splitting point and returns at a different point in time or in space, which actually represents the depth of scan. The time for the reference beam to return represents the desirable depth of penetration of tissue by the sampling beam.

When the reflected beams meet, intensities from respective points with equal time delay form interference. A photodetector detects this interference and converts it into electrical signals. The signals are electronically processed and ultimately displayed, for example, on a computer screen or other monitor.

Obtaining a cross-sectional image of an object involves scanning in both the axial and lateral direction. Typical visual frame rates used in filming moving objects are on the order of 30 Hz. Therefore, to image a moving object, such as a beating heart, for example, a scanner system must be capable of scanning approximately 90,000 data points (assuming 300 data points in both the lateral and axial directions of the object, which is typical for an imaging area of 1X10⁻⁴ mm²) in 1/30 of a second. However, to accomplish lateral scanning, many OCT systems utilize reciprocally-moving mechanical parts to move the beam of light across the object being imaged. These moving parts often cannot move quickly enough to complete a lateral scan in the requisite time required by the visual frame rate. Thus, imaging of moving objects, such as a beating heart, will be incomplete from frame to frame. Additionally, the use of such parts creates other obstacles to achieving effective scanning. For example, the inertia of moving parts, and their acceleration and deceleration, causes a non-uniform speed of scan and a reduced speed of data acquisition. Furthermore, vibrations associated with the moving parts may result in additional electronic noise which negatively affects the resolution of scanned images.

Design and manufacture of an effective scanning device utilizing moving parts in combination with medical tools such as, for example, a catheter, also proves difficult. For instance, it is very difficult to control precisely the motion of parts on a tip of a catheter. Furthermore, moving parts generally require more space, thus resulting in an increase in the overall size of the device. For catheters and other similar medical devices, such an increase in size is undesirable.

Certain scanner systems used in the communications industry exploit the ability of certain materials to change properties (such as refractive index) when subjected to the application of an electromagnetic field. These communications scanner systems typically include an optically transparent prism positioned between electrodes. A light beam passing through the prism will be deflected at certain angles depending on the electromagnetic field created by the electrodes an applied to the prism. Because deflection of the light also is a function of wavelength, optimal performance of these scanners requires a monochromatic (single wavelength) light source. These scanning systems therefore typically utilize lasers, which use light having a narrow bandwidth, as the source of light.

The advantages and purpose of the invention will be set forth in part in the description which follows and, in part, will be obvious from the description or may be learned by practise of the invention. The advantages and purpose of the invention will be realised and attained by means of the elements and combinations particularly pointed out in the appended claims.

According to the present invention there is provided a system for scanning and imaging body tissue, said system comprising:
a catheter for insertion into a body, the catheter having a proximal end and a distal end;
an optical transmitter extending through said catheter from the proximal end to proximate the distal end, said optical transmitter being adapted for transmitting light;
a first lens positioned relative to the optical transmitter for receiving said light from said optical transmitter and transmitting a collimated beam of light;
a prism positioned relative to the first lens to receive said collimated beam of light from said first lens, said prism being made of a material including silica with one or more additives that are chosen such that said material has an index of refraction that varies with changes in an electromagnetic field applied to said prism and remains substantially constant with changes in wavelength of light within a predetermined wavelength range;
means for generating, controlling and applying an electromagnetic field to said prism for controlling the angular deflection of said beam of light passing through said prism for lateral scanning of said body tissue; and
a second lens disposed in an opening at said catheter distal end for focusing light within a predetermined angular range after the light exits said prism.

It is to be understood that both the foregoing general description and the following detailed description are exemplary and explanatory only and are not restrictive of the invention, as claimed.

The accompanying drawings, which are incorporated in and constitute a part of this specification, illustrate the preferred embodiments of the invention and, together with the description, serve to explain the principles of the invention; In the drawings.
Figure 1 is a graph of the index of refraction versus wavelength for crown glass;
Figure 2 is a plan view of a preferred embodiment of an optical scanning system according to the present invention, with a cutaway perspective of a catheter housing several of the scanning system's components; and
Figure 3 is a cutaway perspective view of a portion of the catheter of Figure 2 showing details of the placement of several of the optical scanning system's components within the catheter.

This invention generally pertains to a system for performing imaging of objects, in particular for medical imaging, that overcomes the problems associated with imaging scanners that use mechanically moving parts. For effective performance, particularly in medical imaging, the system must achieve high resolution and scanning rates with a small device.

To accomplish these objectives and to overcome the problems associated with existing devices of this kind, an optical scanning and imaging system for use in medical imaging, according to the present invention incorporates a prism having an index of refraction that changes as a function of an electromagnetic field applied to the prism. By modifying and controlling the electromagnetic field applied to the prism, the index of refraction of the prism, and thus the angular deflection of the light passing through the prism, also can be controlled. The deflected light beam can then be used as the source for an OCT system. This accomplishes imaging of the depth of an object over a lateral dimension of the object without the need for moving components. Thus, a complete scanning of an object can occur in a time period consistent with visual frame rates of approximately 30 Hz to achieve imaging of moving objects, such as, for example, a beating heart, and nonmoving objects. The prism, a means of generating, controlling and applying a electromagnetic field to the prism and a means of transmitting light through the prism are incorporated into a catheter. Incorporating the prism into the medical imaging device virtually eliminates the problems of vibrations and inertia caused by using moving parts with such small devices.

An additional aspect of the present invention focuses on the selection of the prism material to be used in the optical scanning and imaging system. Aside from changing as a function of electromagnetic field, a material's index of refraction also changes with the wavelength of incident light. This becomes problematic when using a prism with an OCT imaging system because such OCT imaging generally requires non-monochromatic light sources having a bandwidth of from 25 to 50 nanometers. Such relatively large bandwidths achieve short coherence, which in turn results in high image resolution. However, when using a large bandwidth light source, the various component wavelengths deflect from the prism at different angles, resulting in a relatively-wide light beam and degrading image resolution.

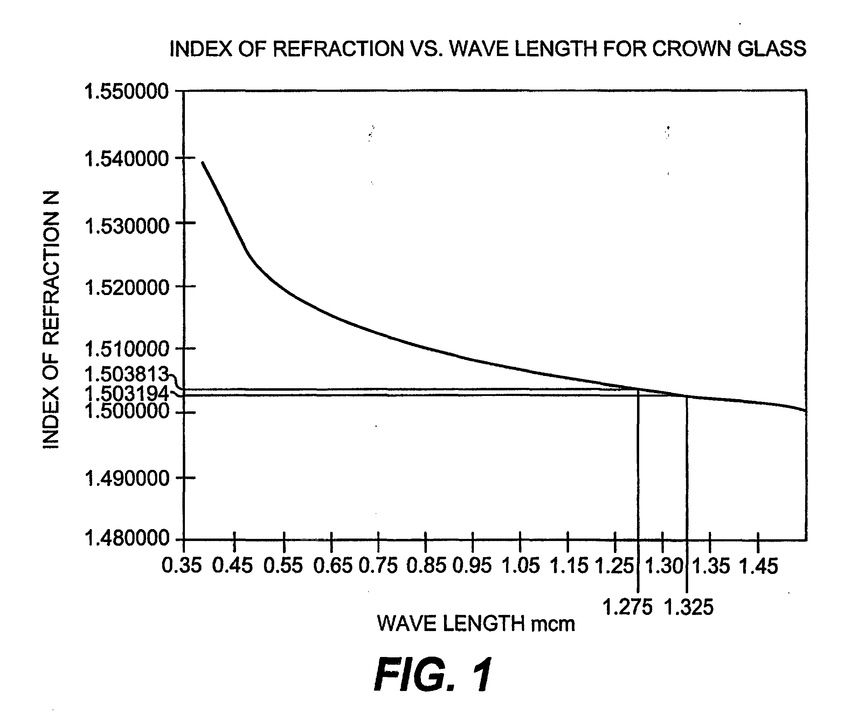

Therefore, according to the present invention, an optical imaging and scanning system incorporates a prism capable of being subject to an electromagnetic field and made of a material which has an index of refraction that remains substantially constant as the wavelength of incident light changes within a defined wavelength range. Furthermore, by carefully selecting the wavelength range of the light source to be used in the optical scanning and imaging system, refractive indices of the prism, as well as other optical components within the system, also can be confined to a relatively narrow range. An exemplary relationship between incident wavelength and index of refraction that is preferred for the prism material of the present invention is shown in Figure 1. Figure 1 illustrates how the index of refraction of crown glass (an example of a glass used in a variety of optics applications) varies with the wavelength of incident light. As shown, the index of refraction changes significantly over smaller ranges of lower wavelength light. However, at ranges of higher wavelength light, for example, between approximately 1275 to 1325 nanometers, the index of refraction remains substantially constant, for example, in a range of approximately 1.503823 to 1.503194.

Additional features that result in the effectiveness of such an imaging device without mechanically moving parts include a coating on optical components to modify and control surface indices of refraction, and optical correcting components, such as a focusing objective lens, to preserve the tightness of focus for a given beam.

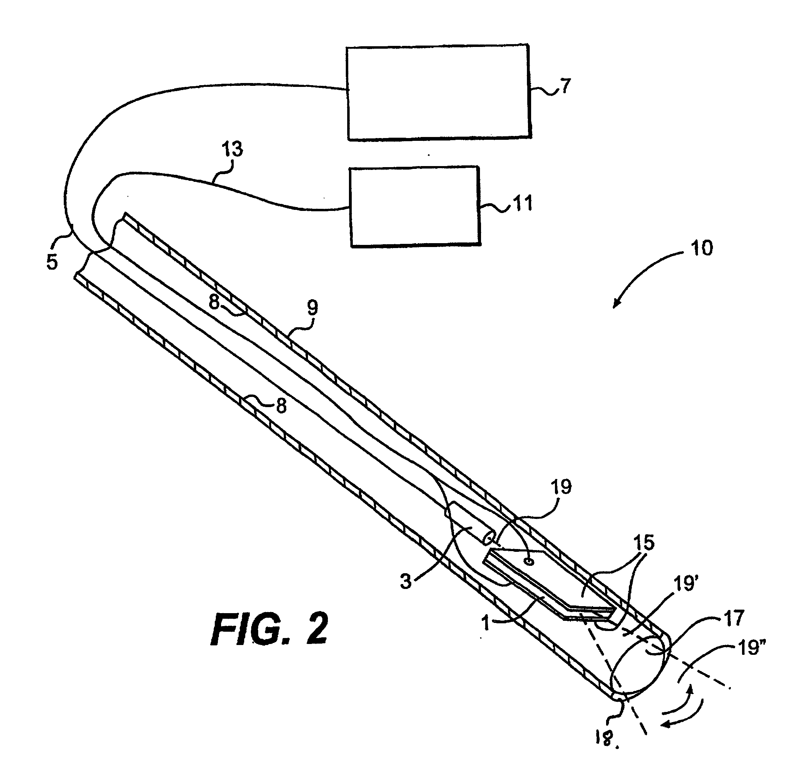

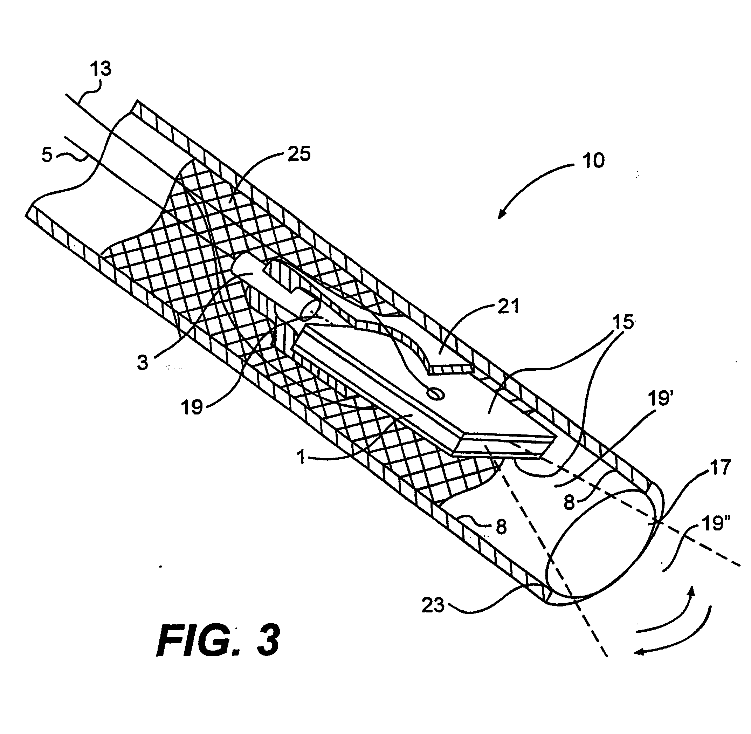

Reference will now be made in detail to the present exemplary embodiments of the invention, examples of which are illustrated in Figures 2 and 3. Wherever possible, the same reference numbers will be used throughout the drawings to refer to the same or like parts.

In accordance with a preferred embodiment of the present invention, an optical scanning and imaging system 10 using both optical and electrical energy is provided. System 10 includes an optical imaging controller 7. Optical imaging controller 7 further includes a light source, a light detector, and processing and imaging electronics (not shown). A controller 7 of suitable components and characteristics would be known to one skilled in the art of optical scanning and imaging. Optical imaging controller 7 connects to an optical transmitter, such as an optical fiber 5. Optical fiber 5 extends between optical imaging controller 7, at one end, to a gradient index (GRIN) lens 3, at a distal end. Optical fiber 5 is preferably of the single mode, polarization-maintaining type which is adapted to conduct optical information. However, it is contemplated that other suitable optical transmitters or fibers for carrying optical signals may be used with the system of the present invention.

A deflecting prism 1 according to an embodiment of the present invention is placed adjacent to and spaced from GRIN lens 3 on a side of GRIN lens 3 opposite to the side to which optical fiber 5 connects. Prism 1 is positioned so that it may receive light emitted from lens 3. According to an aspect of the present invention, deflecting prism 1 is made of a material that varies its index of refraction as a function of an applied electromagnetic field. According to another aspect of the present invention, deflecting prism 1 also is made of a material that has a substantially. constant index of refraction with changes in wavelengths within a specific wavelength range of incident light. One example of such a material that incorporates both of these properties includes silica with additives of, for example, lithium-tantalate (LiTaO₃) or strontium-barium-niobate (SBN). Such a material exhibits a similar index of refraction versus wavelength characteristic as crown glass shown in Figure 1. That is, the general shape of the curve will be similar, although precise numeric values may differ. The various additives that are used in combination with the silica will affect these precise values and should be chosen depending on the desired characteristics, for example the incident wavelength range, of the scanning system.

To apply the electromagnetic field to prism 1, an electrode 15 is placed on each side of prism 1. The electrodes 15 are preferably placed on opposite prism sides that are parallel to the plane of deflection of the light that travels through the prism. A pair of wires 13 each connect at one end to one of the respective electrodes 15. At their other ends, wires 13 connect to a scan controller 11. Scan controller 11 indudes a signal generator and amplifier (not shown). Electrical signals, generated in scan controller 11, travel over wires 13 to electrodes 15 to create an electromagnetic field of controlled strength that is applied to prism 1. It is contemplated that both wires 13 and electrodes 15 are made of copper or silver, or other suitable like material capable of conducting electricity and generally known to those having ordinary skill in the art. Electrodes 15 preferably are in the form of thin plates, however other suitable configurations are contemplated and within the scope of the invention. Moreover, any scan controller of suitable components and characteristics known to one skilled in the art of optical scanning and imaging is within the scope of this invention.

In a preferred embodiment of the present invention, an objective lens 17 is disposed on a side of prism 1 opposite to theside where GRIN lens 3 is disposed. When so disposed, objective lens 17 redirects and focuses a beam of light passing through lens 17 to predetermined angular range. Such a lens also aids in reducing scatter of the ultimate imaging beam in order to produce a system having an overall sharper resolution.

As shown in Figures 2 and 3, the optical scanning and imaging system 10 is disposed within a catheter 9. The scan controller 11 and optical imaging system 7 are located outside of catheter 9. Optical fiber 5 and wires 13 extend from optical imaging system 7 and scan controller 11, respectively, into a proximal end of catheter 9 and through catheter 9 to the remaining components of scanning and imaging system 10 located at a distal end of catheter 9.

Figure 3 illustrates in detail the mounting of optical fiber 5, wires 13, GRIN lens 3, prism 1, and objective lens 17 within catheter 9. A holder 21 secures prism 1 with surrounding electrodes 15 to GRIN lens 3. As shown in the Figure, holder 21 has a substantially U-shaped configuration, with GRIN lens 3 secured to an apex of the U while the legs of the U clamp around outer surfaces of electrodes 15 to hold prism 1. Holder 21, thus, is configured to maintain a predetermined distance between GRIN lens 3 and prism 1. Holder 21 preferably is made from a material that exhibits high rigidity, strength, and insulating properties, such as, for example, polycarbonate or other suitable like material. While Figure 3 shows holder 21 having a U-shaped configuration, other configurations are contemplated as long as they allow for firm securing of prism 1. electrodes 15 and GRIN lens 3 relative to each other.

A fixing member 25, preferably in the form of potting compound, secures the entire assembly, including optical fiber 5, GRIN lens 3, electrical wires 13, electrodes 15, and holder 21, within catheter 9. Member 25 preferably fills substantially the entire space between the various components and inner walls 8 of catheter 9 and extends along a length of catheter 9 from approximately a distal end of prism 1 to slightly past a proximal end of GRIN lens 3. It is contemplated to use a potting compound such as silicon rubber or epoxy for member 25. However other suitable like materials also may be used and would be within the scope of the present invention:

Objective lens 17 is secured to the distal opening 18 of catheter 9 using an adhering member 23. Member 23 Is preferably in the form of optical adhesive having similar optical properties as the incident beam of light used in conjunction with optical scanning and imaging system 10, while also being capable of sealing the inside of catheter 9 from the penetration of moisture. An example of such an optical adhesive includes an adhesive called "Norland Optical Adhesive 61", supplied by Norland Products, Inc. However, other epoxies or suitable like optical adhesives having the desired properties can be used and would be within the scope of the present invention.

A preferred embodiment of the combined optical scanning system 10 and catheter 9 has the following dimensions so as to be compatible for use with typical endoscopes. Catheter 9 has an outside diameter of approximately 2.7 mm, an inside diameter of approximately 2.3 mm and a wall thickness of approximately 0.2 mm. Deflecting prism 1 with attached electrodes 15 is approximately 20 mm long, approximately 2.2 mm wide and approximately 0.5 mm thick. GRIN lens 3 generally has a cylindrical configuration with a diameter of approximately 0.5 mm and a length of approximately 3 to 5 mm. The overall length of the system from a proximal end of GRIN lens 3 to a distal end of objective lens 17 is approximately 40 mm, including gaps between GRIN lens 3 and prism 1 and prism 1 and objective lens 17. These dimensions are meant to be exemplary only. It is contemplated that the various components' dimensions, as well as the overall system dimensions, may vary according to the precise procedure the system is used to perform. For instance, it will be appreciated by persons having ordinary skill in the art that the gap between GRIN lens 3 and prism 1 is not critical and can be as little as zero since a collimated beam of light exits GRIN lens 3. The gap between prism 1 and objective lens 17 depends on the focal length of objective lens 17 which is designed according to actual deflection angular range exhibited by prism 1. and the desired range of angular deflection exiting objective lens 17. The thickness of objective lens 17 also. may be determined by desired performance.characteristics.

System 10 works in the following manner. Light is generated by the light source within optical imaging system 7. The wavelength range emitted by the light source is selected as a function of the material used for prism 1. That is, the incident wavelengths must be selected within the range for which the prism material exhibits, a relatively constant index of refraction over the entire range. Choosing relatively high wavelengths for the incident light allows for greater penetration of the depth of the object Thus, it is preferable to select a material for prism 1 that has substantially constant indices of refraction over ranges of relatively high wavelengths.

The radiated light from system 7 is polarized in a plane perpendicular to a plane of scanning and travels along optical fiber 5 and through GRIN lens 3. From GRIN lens 3, the light exits as a collimated beam 19 and enters prism 1. Prism 1 deflects collimated beam of light 19 in an angular direction in the scanning plane. The deflected light beam 19' then enters objective lens 17 which focuses and redirects the beam within a predetermined angular range in order to achieve sharper image resolution.

Light 19" exiting objective lens 17 is then directed to the object to be imaged. The reflected light from the object then enters objective lens 17. goes back through prism 1, GRIN lens 3 and optical fiber 5 to the optical imaging controller 7, where it is processed and converted into an image, as described earlier with respect to OCT systems generally. The processed image is then displayed on a computer monitor or other suitable, like display means.

An aspect to the operation of optical scanning and imaging system 10 includes controlling the angle of deflection of light beam 19' exiting deflecting prism 1. As previously discussed, prism 1 is made of a material that has a Varying index of refraction with changes in an applied electromagnetic field. Thus, the refractive index of prism 1, as well as the angular deflection of beam 19', can be controlled and modified by controlling and modifying the amplitude and frequency of the electromagnetic field generated between electrodes 15. The signal generator and amplifier of scan controller 11 control the frequency and amplitude of the electromagnetic field, and can be either manually controlled by a user or automatically controlled using systems generally known to those stalled in the art.

It will be apparent to those skilled in the art from consideration of the specification and practice of the invention disclosed herein that various modifications and variations can be made in the optical scanning and imaging system according to the present invention. For example, preferred materials for component parts of the system, including the prism, have been suggested in the specification, but other materials having similar properties could be utilized as well. Also, the sizes and shapes of the various components, including the GRIN lens, the prism, the electrodes and the objective lens of the system may differ from one embodiment to the next depending on, for example, the medical procedure to be performed. Various changes in size and shape may also be necessary depending on the types of objects desired to be imaged and the environments in which the objects are located. Additionally, it is contemplated to use surface coatings on various optical components within the system to alter various properties of those components, including, for example, the index of refraction. Selection of coatings for the desired properties would be obvious to persons having ordinary skill in the art.

## Claims

1. A system for scanning and imaging body tissue, said system comprising:
a catheter (9) for insertion into a body, the catheter having a proximal end and a distal end (18);
an optical transmitter (5) extending through said catheter from the proximal end to proximate the distal end, said optical transmitter being adapted for transmitting light;
a first lens (3) positioned relative to the optical transmitter (5) for receiving said light from said optical transmitter and transmitting a collimated beam (19) of light;
a prism (1) positioned relative to the first lens to receive said collimated beam (19) of light from said first lens, said prism being made of a material including silica with one or more additives that are chosen such that said material has an index of refraction that varies with changes in an electromagnetic field applied to said prism and remains substantially constant with changes in wavelength of light within a predetermined wavelength range;
means (11, 13, 15) for generating, controlling and applying an electromagnetic field to said prism for controlling the angular deflection of said beam of light (19) passing through said prism (1) for lateral scanning of said body tissue; and
a second lens (17) disposed in an opening (18) at said catheter distal end for focusing light within a predetermined angular range after the light exits said prism.

2. A system as claimed in claim 1, wherein said material comprises silica with additives of lithium tantalate or strontium-barium-niobate.

3. A system as claimed in claim 1 or claim 2, wherein said optical transmitter (5) includes an optical fibre.

4. A system as claimed in claim 1, claim 2 or claim 3, wherein said first lens is a gradient index lens.

5. A system as claimed in any of claims 1 - 4, further comprising an optical imaging . controller (7) coupled to the optical transmitter (5) for supplying incident light to said optical transmitter and receiving reflected light from the tissue being imaged.

6. A system as claimed in any of claims 1 -5, further comprising a mounting plate (21) for holding the first lens (3) and the prism (1) in a fixed position relative to each other within said catheter (9).

7. A system as claimed in any of claims 1 - 6, wherein said optical transmitter (5), said first lens (3) and said prism (1) are secured within said catheter (9) by a fixing member (25).

8. A system as claimed in claim 7, wherein said fixing member (25) includes potting compound.

9. A system as claimed in any of claims 1-8, wherein said second lens (17) is secured to said catheter opening (18) by an adhesive such that said second lens (17) and said optical adhesive seal said catheter opening (18).

10. A system as claimed in any of claims 1.9, further comprising electrodes (15) positioned relative to the prism (1) and configured to apply said electromagnetic field to said prism.

11. A system as claimed in claim 10, wherein first and second electrodes (15) are positioned on respective first and second opposite sides of the prism (1) that are parallel to a plane of deflection of the light (19') exiting the prism.

12. A system as claimed in claim 10 or 11, further comprising a scan controller (11) for controlling and modifying said electromagnetic field to control and modify said index of refraction of said minim

13. A system as claimed in claim 12, wherein said scan controller (11) is coupled to said electrodes (15) by wires (13).

## Patentansprüche

1. System zum Abtasten und Abbilden von Körpergewebe, wobei das System aufweist:
einen Katheter (9) zum Einführen in einen Körper, wobei der Katheter ein proximales Ende und ein distales Ende (18) aufweist;
einen optischen Transmitter (5), welcher sich durch den Katheter hindurch von dem proximalen Ende erstreckt, um sich dem distalen Ende anzunähern, wobei der optische Transmitter in der Lage ist, Licht zu übertragen;
eine erste Linse (3), welche relativ zu dem optischen Transmitter (5) positioniert ist, um Licht von dem optischen Transmitter zu empfangen und um einen parallel gerichteten Lichtstrahl (19) zu übertragen;
ein Prisma (1), welches relativ zu der ersten Linse positioniert ist, um den parallel gerichteten Lichtstrahl (19) von der ersten Linse zu empfangen, wobei das Prisma aus einem Material hergestellt ist, welches Silizium mit einem oder mehreren Additiven umfasst, welche ausgewählt sind derart, dass das Material einen Brechungsindex aufweist, der mit Änderungen in einem elektromagnetischen Feld variiert, welches auf das Prisma ausgeübt wird, und welcher bei Änderungen in der Wellenlänge des Lichts innerhalb eines vorbestimmten Wellenlängenbereichs im Wesentlichen konstant bleibt;
Mittel (11, 13, 15) zum Erzeugen, Steuern und Ausüben eines elektromagnetischen Felds auf das Prisma zum Steuern der winkelmäßigen Ablenkung des Lichtstrahls (19), welcher durch das Prisma (1) zum lateralen Abtasten des Körpergewebes wandert; und
eine zweite Linse (17), welche in einer Öffnung (18) an dem distalen Ende des Katheters angeordnet ist zum Fokussieren von Licht innerhalb eines vorbestimmten Winkelbereichs, nachdem das Licht aus dem Prisma ausgetreten ist.

2. System nach Anspruch 1, bei welchem das Material Silizium mit Additiven von Lithiumtantalat oder Strontium-Barium-Niobat aufweist.

3. System nach Anspruch 1 oder Anspruch 2, bei welchem der optische Transmitter (5) einen Lichtleiter umfasst.

4. System nach Anspruch 1, Anspruch 2 oder Anspruch 3, bei welchem die erste Linse eine Gradientenlinse ist.

5. System nach einem der Ansprüche 1 bis 4, weiter aufweisend eine optische Abbildungssteuerung (7), welche mit dem optischen Transmitter (5) gekoppelt ist, um dem optischen Transmitter ein einfallendes Licht zur Verfügung zu stellen und um von dem Gewebe, welches abgebildet wird, reflektiertes Licht zu empfangen.

6. System nach einem der Ansprüche 1 bis 5, weiter ausweisend eine Montageplatte (21) zum Halten der ersten Linse (3) und des Prismas (1) in einer fixierten Position relativ zueinander im Inneren des Katheters (9).

7. System nach einem der Ansprüche 1 bis 6, bei welchem der optische Transmitter (5), die erste Linse (3) und das Prisma (1) im Inneren des Katheters (9) durch ein Befestigungselement (25) befestigt sind.

8. System nach Anspruch 7, bei welchem das Befestigungselement (25) eine Vergießmasse umfasst.

9. System nach einem der Ansprüche 1 bis 8, bei welchem die zweite Linse (17) an der Öffnung (18) des Katheters durch einen Klebstoff derart befestigt ist, dass die zweite Linse (17) und der optische Klebstoff die Öffnung (18) des Katheters abdichtet.

10. System nach einem der Ansprüche 1 bis 9, weiter aufweisend Elektroden (15), welche relativ zu dem Prisma (1) positioniert sind und welche konfiguriert sind, um das elektrische Feld an das Prisma anzulegen.

11. System nach Anspruch 10, bei welchem erste und zweite Elektroden (15) auf jeweils ersten und zweiten gegenüberliegenden Seiten des Prismas (1) positioniert sind, welche parallel zu einer Ebene einer Ablenkung des Lichts (19'), welches aus dem Prisma austritt, sind.

12. System nach Anspruch 10 oder 11, weiter aufweisend eine Abtaststeuerung (11) zum Steuern und Modifizieren des elektromagnetischen Felds, um den Brechungsindex des Prismas zu steuern und zu modifizieren.

13. System nach Anspruch 12, bei welchem die Abtaststeuerung (11) mit den Elektroden (15) durch Drähte (13) gekoppelt ist.

## Revendications

1. Système de balayage et de formation d'image d'un tissu corporel, ledit système comprenant :
- un cathéter (9) pour l'insertion dans un corps, le cathéter ayant une extrémité proximale et une extrémité distale (18) ;
- un transmetteur optique (5) s'étendant en traversant ledit cathéter depuis l'extrémité proximale jusqu'au voisinage de l'extrémité distale, ledit transmetteur optique étant adapté pour transmettre de la lumière ;
- une première lentille (3) par rapport au transmetteur optique (5) pour recevoir ladite lumière provenant dudit transmetteur optique et pour transmettre un faisceau collimaté (19) de lumière ;
- un prisme (1) positionné par rapport à la première lentille pour recevoir ledit faisceau collimaté (19) provenant de ladite première lentille, ledit prisme étant réalisé en un matériau comprenant de la silice avec un ou plusieurs additifs qui sont choisis de telle sorte que ledit matériau a un indice de réfraction qui varie en fonction de modifications dans le champ électromagnétique appliqué audit prisme et qui reste sensiblement constant lors des changements dans les longueurs d'onde de la lumière dans une plage de longueurs d'ondes prédéterminée ;
- des moyens (11, 13, 15) pour générer, commander et appliquer un champ électromagnétique audit prisme pour commander la déflection angulaire dudit faisceau de lumière (19) traversant ledit prisme (1) pour balayer latéralement ledit tissu corporel ; et
- une seconde lentille (17) disposée dans un orifice (18) à ladite extrémité distale de cathéter pour focaliser la lumière à l'intérieur d'une plage angulaire prédéterminée après la sortie de la lumière hors dudit prisme.

2. Système selon la revendication 1, dans lequel ledit matériau comprend de la silice avec des additifs de tantalate de lithium ou de niobate de barium et de strontium.

3. Système selon la revendication 1 ou la revendication 2, dans lequel ledit transmetteur optique (5) comprend une fibre optique.

4. Système selon la revendication 1, la revendication 2 ou la revendication 3, dans lequel ladite première lentille est une lentille à gradient d'index.

5. Système selon l'une quelconque des revendications 1 à 4, comprenant en outre un contrôleur de génération d'images optiques (7) couplé au transmetteur optique (5) pour fournir la lumière incidente audit transmetteur optique et pour recevoir la lumière réfléchie provenant du tissu dont on génère l'image.

6. Système selon l'une quelconque des revendications 1 à 5, comprenant en outre une plaque de montage (21) pour tenir la première lentille (3) et le prisme (1) en une position fixe l'un part par rapport à l'autre à l'intérieur dudit cathéter (9).

7. Système selon l'une quelconque des revendications 1 à 6, dans lequel ledit transmetteur optique (5), ladite première lentille (3) et ledit prisme (1) sont fixés à l'intérieur dudit cathéter (9) par un élément de fixation (25).

8. Système selon la revendication 7, dans lequel ledit élément de fixation (25) comprend un composé d'empotage.

9. Système selon l'une quelconque des revendications 1 à 8, dans lequel ladite seconde lentille (17) est fixée audit orifice (18) de cathéter par un adhésif de telle sorte que ladite seconde lentille (17) et ledit adhésif optique obturent de façon étanche ledit orifice (18) de cathéter.

10. Système selon l'une quelconque des revendications 1 à 9, comprenant en outre des électrodes (15) positionnées par rapport au prisme (1) et configurées pour appliquer ledit champ électromagnétique audit prisme.

11. Système selon la revendication 10, dans lequel des première et seconde électrodes (15) sont positionnées sur des premier et second côtés opposés respectifs du prisme (1) qui sont parallèles à un plan de déflection de la lumière (19') émergeant du prisme.

12. Système selon la revendication 10 ou la revendication 11, comprenant en outre un contrôleur de balayage (11) pour commander et modifier ledit champ électromagnétique pour commander et modifier ledit indice de réfraction dudit prisme.

13. Système selon la revendication 12, dans lequel ledit contrôleur de balayage (11) est couplé auxdites électrodes (15) par des conducteurs (13),
